# EUROPEAN PATENT SPECIFICATION

(11) **EP 1 428 476 B1**
(45) Date of publication and mention of the grant of the patent: **09.01.2008**
(21) Application number: 03256669.7
(22) Date of filing: 22.10.2003
(51) Int. Cl.: A61B 10/00

(54) **Biopsy device handle assembly**
Handgriffanordnung für eine Biopsievorrichtung
Ensemble poignée pour un dispositif de biopsie

(30) Priority: 22.10.2002 US 277313
(43) Date of publication of application: 16.06.2004
(73) Proprietor: Allegiance Corporation, McGaw Park, Illinois 60085-6787 (US)
(72) Inventor: Krueger, John, Milwaukee, Wisconsin 53222 (US); Groenke, Gregory C., Gurnee, Illinois 60031 (US)
(74) Representative: Dee, Ian Mark

(56) References cited:
- EP-A- 0 754 431
- US-A- 4 664 381
- US-A- 4 793 363
- US-A- 5 385 151
- US-B1- 6 312 394

## Description

### FIELD OF THE INVENTION

The invention relates to biopsy devices useful during medical procedures. In particular, the invention pertains to a handle assembly for use in conjunction with bone biopsy devices.

### BACKGROUND OF THE INVENTION

Biopsy devices which can be used to obtain tissue samples from harder tissues, such as bone, are well known in the art. Bone biopsy devices can contain a structure which can penetrate through the cortex of bone and obtain a "core" sample of the harder bone tissue and softer marrow tissue within from the sampling site. One common structure which is present on certain bone biopsy devices is a cannula and inner stylet or trocar assembly. In these devices, the stylet or trocar is initially secured within an outer cannula and has a sharpened tip which is used to bore into the bone to access the sampling site. Another variety of biopsy devices involve those which use aspiration to obtain liquid samples from a tissue site, such as contents found in bone marrow.

A variety of devices having the stylet-and-cannula structure have been developed. Furthermore, a variety of approaches have also been developed in an attempt to make the use of such devices more accurate and comfortable for the practitioner. The comfort and ergonomics of such devices is especially important given the pressure and forces, both rotationally as well as longitudinally, which is applied by the user to the device during the penetration and sampling steps. Various biopsy device handle configurations are described by Ausherman et al. U.S. Patent No. 4,793,363, Mehl U.S. Patent No. 4,469,109, Tretinyak U.S. Patent No. 4,630,616, Mathis et al. U.S. Patent No. 6,221,029, Fleming, III et al. U.S. Patent No. 6,302,852, Fleming, III U.S. Patent No. 6,312,394, and Lee U.S. Patent No. 4,655,226.

One problem associated with bone biopsy devices is the development of a biopsy device which contains both advantageous structural features capabilities as well as enhanced user comfort. A further problem in the development of bone biopsy devices is improving their sampling structures while at the same time reducing the trauma to the sampling site for the patient.

These attributes have been especially difficult to balance in those biopsy device handle assemblies, namely bone tissue sampling devices, wherein the handle components are designed to reversibly separate as part of their sampling operation. There is a need in the medical field for improved bone biopsy devices which contain separable components, are easy to operate, and furthermore enhance the comfort of their use to both the user as well as the patient.

### SUMMARY OF THE INVENTION

The invention provides a handle assembly for biopsy devices which comprise a separable outer cannula and stylet component structure. More specifically, the invention involves an improved handle assembly for such devices which permits securing and removal of the stylet from the inside of the outer cannula by virtue of separating the handle components. It has been discovered that a handle assembly can be structured to not only perform the functions of a bone biopsy device, but that the handle can be structured to permit separation of a stylet from the interior of an outer cannula by separating handle components while at the same time enhancing the comfort, maneuverability, and precision to the user during its operation. The handle assembly of the invention facilitates control and accuracy of the biopsy device during its use, while at the same time affords comfort to the user during its operation. Furthermore, it has been discovered that the handle assembly of the invention can be "universal" in that the same basic handle assembly structure and its basic components can be used to make bone tissue core sampling structures or, alternatively, bone marrow aspiration structures.

The invention provides a handle assembly for use with a biopsy device according to claim 1 and a biopsy device according to claim 13.
In one embodiment, the assembled handle comprises four concave arcuate indentations located on the side of the handle and defining corresponding arcuate regions of the outer perimeter of the top portion upper and lower surfaces and the bottom portion upper and lower surfaces.

In a preferred embodiment, the concave arcuate indentations are positioned such that each of the two longer side regions of the assembled handle comprises two concave indentations positioned apart from one another, and each concave indentation is positioned opposite a corresponding concave indentation on the opposite side of the handle.

In an even more preferred embodiment, the circumscribing edges of both the top portion and bottom portion at the peripheral juncture at which they mate are rounded, curved and smooth. Thus, the central region of the handle assembly is devoid of sharp edges. Furthermore, the entire exterior hand-contacting surface of the handle assembly can be substantially smooth and rounded.

The handle assembly of the invention can be used in conjunction with a variety of biopsy device structures that include an outer cannula and a removable inner stylet positioned within the outer cannula. In one embodiment, the handle assembly of the invention is part of a bone tissue core sampling device and is attached to an outer cannula and inner stylet structured for such. In another embodiment, the handle assembly is part of a bone marrow aspiration sampling device, wherein the bottom portion of the handle assembly can further comprise an adjustable depth guide assembly that can be removably attached to the bottom portion of the handle assembly.

### BRIEF DESCRIPTION OF THE DRAWINGS

The following figures further illustrate the invention and are not intended to be construed in a limiting manner:
**Figure** 1 is an angled side view perspective of a biopsy device with the handle assembly according to one embodiment of the invention.
**Figure 2** is a front upright view of a biopsy device with the handle assembly according to one embodiment of the invention.
**Figure 3** is a back upright view of a biopsy device with the handle assembly according to one embodiment of the invention.
**Figure 4** is a left side view of a biopsy device with the handle assembly according to one embodiment of the invention.
**Figure 5** is a right side view of a biopsy device with the handle assembly according to one embodiment of the invention.
**Figure 6** is a top view of the biopsy device with the handle assembly according to one embodiment of the invention.
**Figure 7** is a bottom view of the biopsy device with the handle assembly according to one embodiment of the invention.
**Figure 8** is an exploded view of the biopsy device and handle assembly structured for core sampling bone and marrow tissue showing the individual component parts separated from one another according to one embodiment of the invention.
**Figure 9** is a disassembled view of the biopsy device and handle assembly structured for core sampling bone and marrow tissue showing the top portion of the handle containing a stylet attached thereto separated from the bottom portion and remainder of the device.
**Figure 10** is an exploded view of the biopsy device and handle assembly structured for aspiration sampling showing the individual component parts separated from one another according to one embodiment of the invention.
**Figure 11** is a disassembled view of the biopsy device and handle assembly structured for aspiration sampling showing the top portion of the handle containing the stylet attached thereto separated from the bottom portion and remainder of the device.
**Figure 12** is a combined view of the lower surface of the top handle portion and the upper surface of the bottom handle portion.

### DETAILED DESCRIPTION OF THE INVENTION

The term "indentation" as used herein within the context of "concave arcuate indentation" is meant to describe a smooth, curved recess on the side of the handle having the general dimensions which correspond to a portion of the natural curvature of the circumference of a human finger.

The term "arcuate" as used to define the overall shape of the handle portion of the invention is intended to indicate that the assembled handle components have an arcuate configuration wherein both the upper and lower handle components are correspondingly arched. Specifically, the upper surface arc bows outward and the lower surface arc bows inward.

The biopsy device handle assembly of the invention is structured to both functionally and ergonomically enhance the use and operation of the biopsy device. The inventive combination of features described herein, including the structure and configuration of the handle portion, afford the biopsy device with ease and comfort of use and operation, while also facilitating the accuracy and precision of its use during a medical procedure. Given the pressure and forces which must be exerted by the practitioner's hand to obtain a bone tissue sample from a patient, as well as the resulting trauma to the sampling site, the handle assembly features of the invention and their associated advantages are important to both the practitioner and the patient.

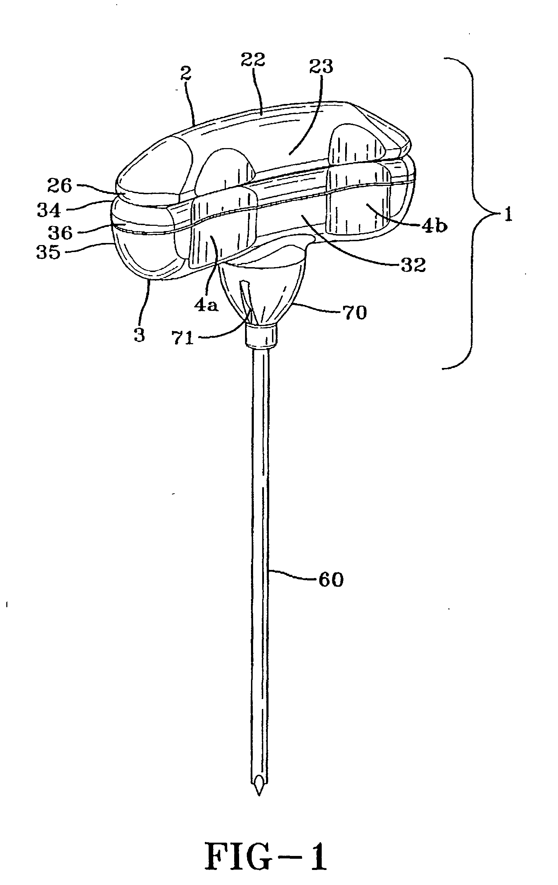

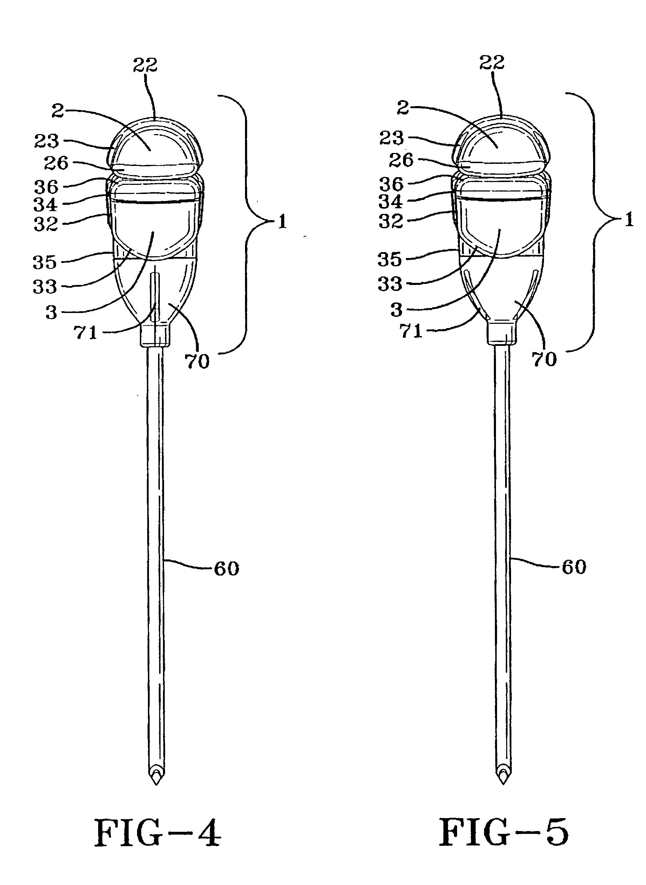

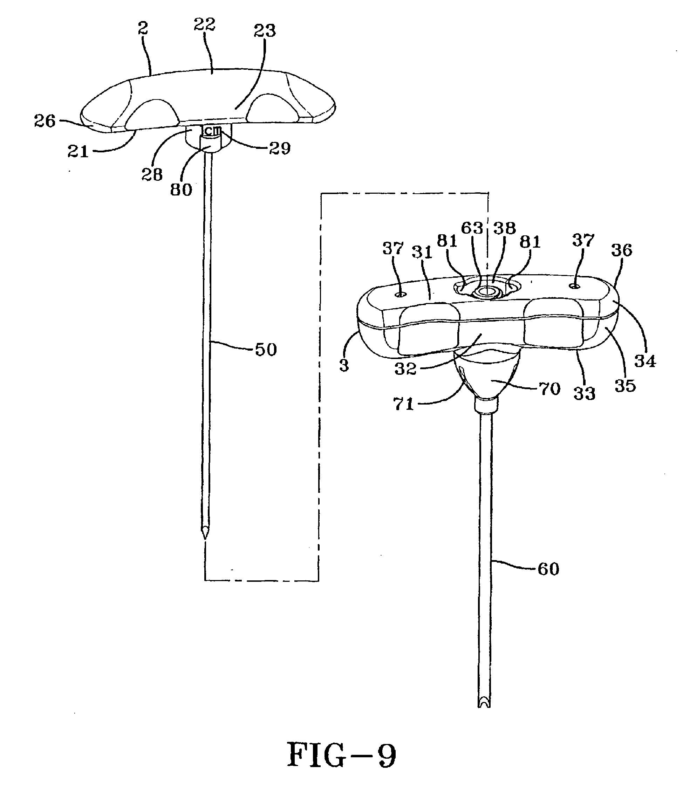

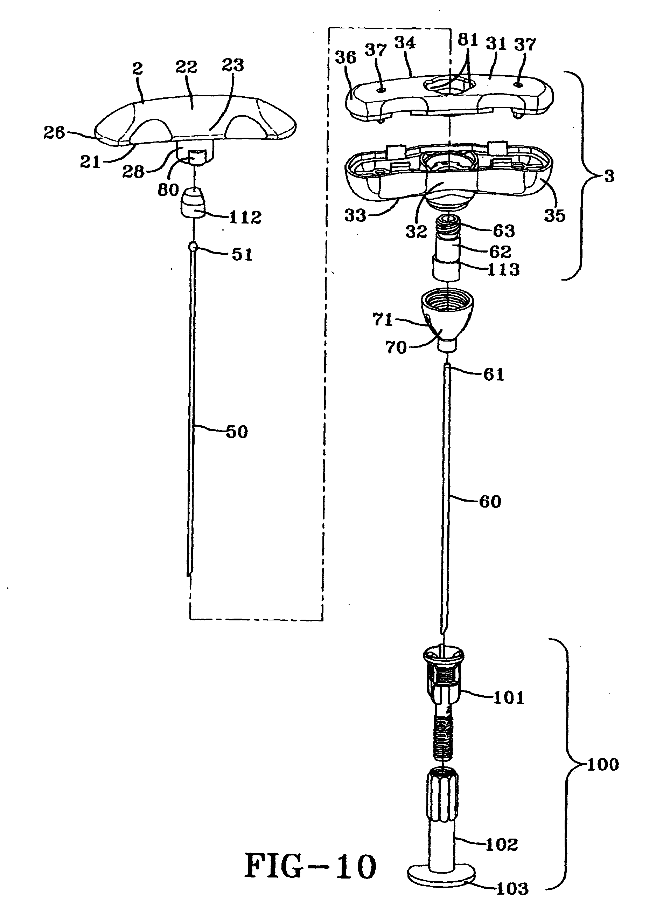

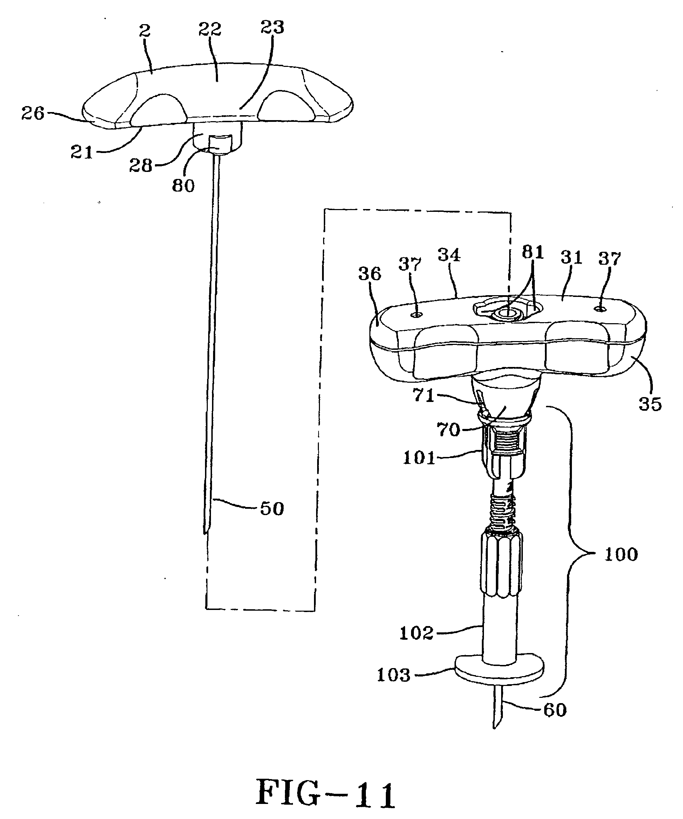

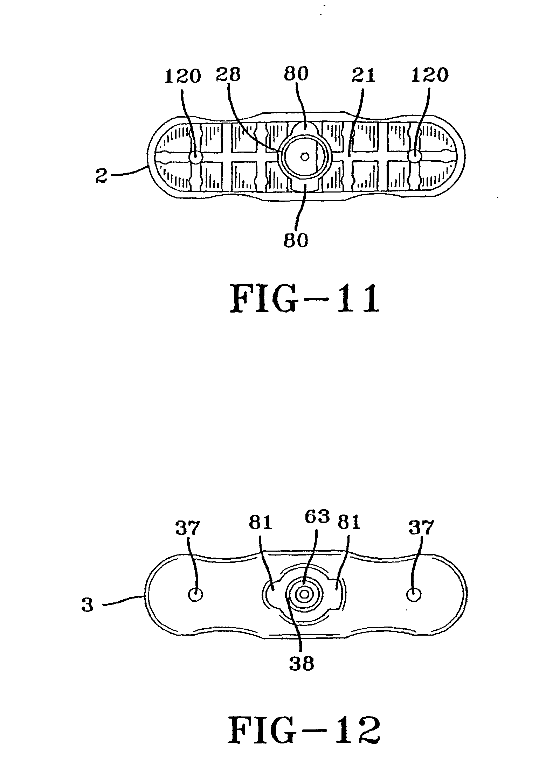

The basic components of the handle assembly 1 of the invention are illustrated as part of a bone biopsy core sampling device in Figures 1 through 9. Figures 10 and 11 illustrate the handle assembly 1 of the invention as part of a bone marrow aspiration device. In any case, the handle assembly 1 can be employed as part of a biopsy device that comprises an outer cannula and removable inner stylet assembly and which is adapted for manual operation. The handle assembly 1 permits securing and removal of the stylet from the inside of the outer cannula by virtue of separating the handle components, as illustrated in Figures 9 and 11. As can be seen from these figures, the handle assembly comprises at least two separable handle components that together form a generally elongated arcuate handle. Unless indicated otherwise, it will be understood that the description of the handle assembly of the invention can apply to both a bone tissue core sampling device and bone marrow aspiration device that contain the outer cannula and removable inner stylet components.

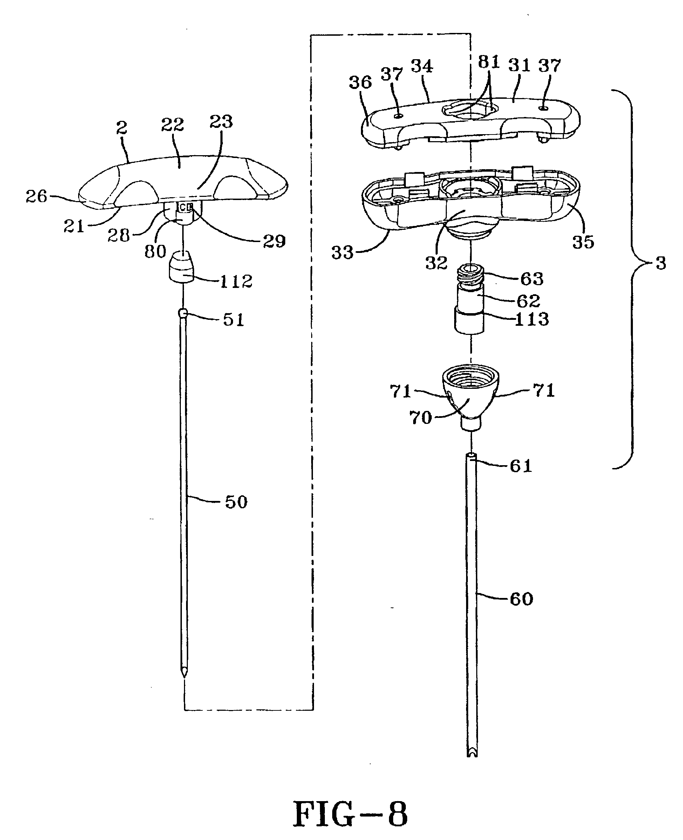

The handle assembly 1 components includes a top portion 2 and bottom portion 3. The top portion lower surface 21 mates with the bottom portion upper surface 31 to form an assembled handle comprising a generally arcuate handle wherein the outward bow or curve is convex at the top portion and the inward bow or curve is concave at the bottom portion. The top portion 2 comprises an upper surface 22, circumscribing side surface 23, and lower surface 21. The bottom portion 3 comprises an upper surface 31, circumscribing side surface 32, and lower surface 33. Referring to Figures 8 and 10, for manufacturing purposes the bottom portion 3 can itself can be formed by the coupling of a first bottom portion half 34 and a second bottom portion half 35 which are affixed to one another. In the final manufactured form of the handle assembly 1, these halves are not readily separable and remain in fixed relationship during the use of the device.

The exterior of the handle assembly has a symmetrical exterior appearance relative to a central axis running from the top to the bottom of the handle. Accordingly, a specific or particular gripping hand arrangement by the user is not required.

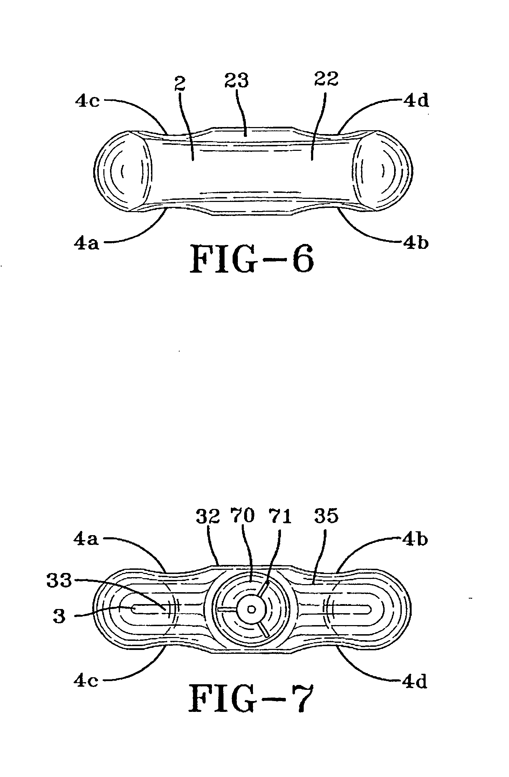

The handle assembly 1 can further comprise four concave indentations 4a, 4b, 4c and 4d, located on the longer two sides of the handle and defining corresponding inwardly curved regions of the outer perimeter of the handle assembly 1. In a preferred embodiment and as depicted in Figures 6 and 7, concave arcuate indentations are positioned such that each of the two longer side regions of the assembled handle comprises two concave indentations positioned apart from one another, and each concave indentation is positioned opposite a corresponding concave indentation on the opposite side of the handle. In accordance with the invention, the concave indentations are present on each of the elongate sides of the handle assembly and extend contiguously from the sides of the top portion 2 to the sides of the bottom portion 3. Thus, the concave indentations are part of the definition of the sides of the top and bottom components of the handle assembly so as to form a substantially smooth transition between the handle surfaces for the user's fingers.

In an even more preferred embodiment, the circumscribing edges 26 and 36 of both the top portion 2 and bottom portion 3, respectively, at the peripheral juncture at which they mate are rounded, curved and smooth. Thus, the central region of the handle assembly is devoid of sharp and relatively uncomfortable edges. This feature significantly contributes to the comfort of handling and using the device.

The handle assembly 1 of the invention can be used in conjunction with a variety of biopsy device structures, including those comprising an outer cannula and a removable inner stylet positioned within the outer cannula. Referring now to Figures 9 and 11, the inner stylet 50 is affixed to the bottom surface 21 of the top portion 2 of the handle assembly 1, whereas the outer cannula 60 is secured to the bottom portion 3 of the handle assembly. In a preferred embodiment, the juncture of the outer cannula 60 and bottom portion 3 of the handle assembly 1 is covered by a cap 70 adapted to removably engage to the lower surface 33 of the bottom portion 3 of the handle and adapted to accommodate a portion of the outer cannula 60 running therethrough. The cap 70 can have an overall conical shape. A variety of removable engagement structures can be used with the cap provided that such structures can reversibly join the cap to the bottom portion of the handle. One engagement structure which can be used is a threaded structure as illustrated in Figures 8 and 10. The exterior surface of the cap 70 can further comprise a corresponding securing structure, which can be configured to receive tools or the proximal end of a depth guide, for example. The figures depict a plurality of elongate grooves 71 intermittently positioned around the exterior of the cap 70.

In order to enhance the interfitting between the top portion 2 and bottom portion 3 of the handle assembly 1 and indicate proper alignment, the lower surface 21 of the top portion 2 and the upper surface 31 of the bottom portion 3 can further comprise cooperating interfitting alignment structures, such as a nub-and-dint structure as illustrated. Referring now to Figure 12, the lower surface 21 of the top portion 2 can contain a pair of nubs 120 which are positioned to cooperate with and interfit into a corresponding pair of dints 37 located on the upper surface 31 of the bottom portion 3 as shown in Figures 8, 9, 10 and 11. The inverse arrangement is also possible. A variety of other such interfitting alignment structures are also possible provided they indicate that the predetermined alignment relationship between the top and bottom portions while permitting rotational separation between the top portion and bottom portion.

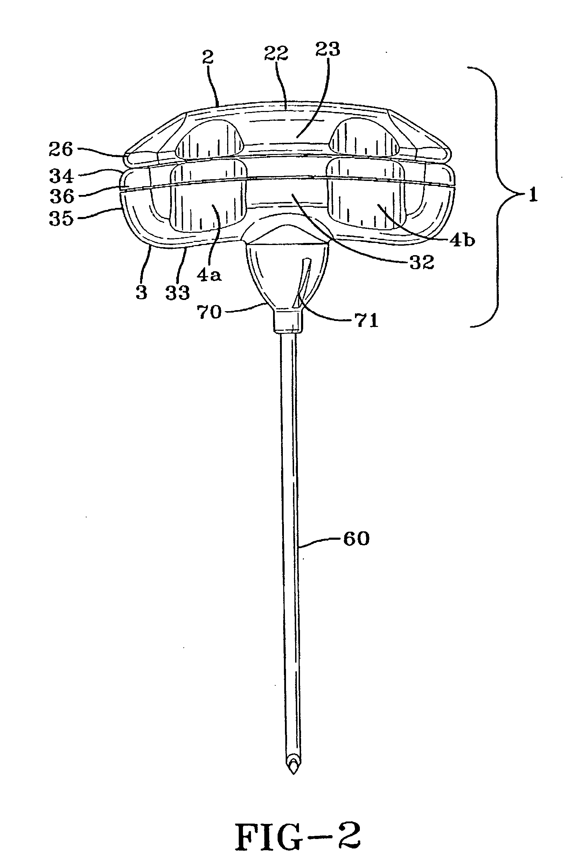

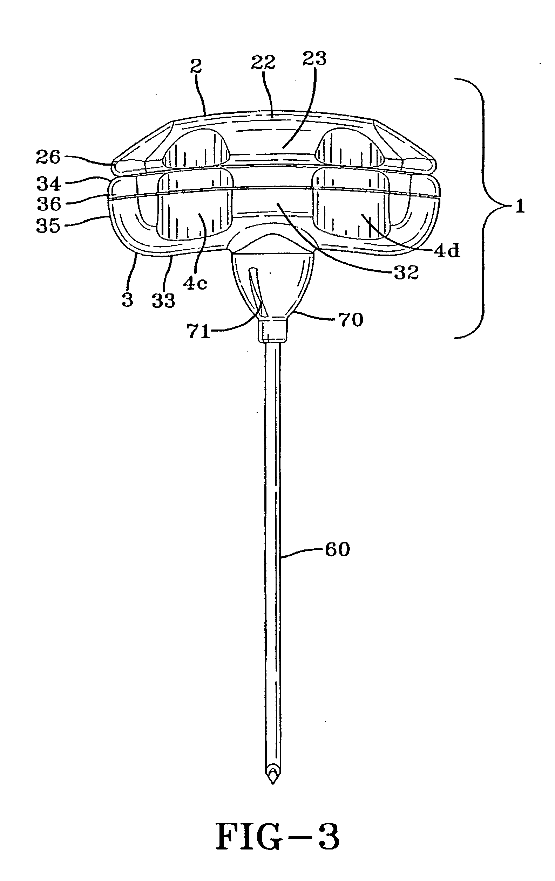

Referring to Figures 2 and 3, the upper surface 22 of the top portion 2 of the handle assembly 1 comprises the outward convex arc of the generally arcuate handle configuration. The lower surface 21 of the top portion 2 further comprises an inner stylet hub 28 adapted to interfit into a recess 38 located on the upper surface 31 of the bottom portion 3, and into which the proximal end 51 of the inner stylet 50 is secured. Between the proximal end 51 of the inner stylet 50 and the inner inner stylet hub 28 can be a hub fitting 112 (see Figures 8 and 10), which enhances securing the inner stylet 50 within the inner stylet hub 28 of the top handle portion 2. The separation of the top portion 2 of the handle assembly 1 from the bottom portion 3 likewise withdraws the inner stylet 50 from the interior of the outer cannula 60, which itself is secured to the bottom portion 3.

In a further embodiment, the inner stylet hub 28 of the top portion 2 and bottom portion recess 38 comprise cooperating orientation and alignment structures which control the fitting arrangement between the top portion and bottom portion of the handle assembly. In a preferred embodiment, the orientation and alignment structures are asymmetrical so as to permit a single predetermined fitting arrangement and rotation direction to interfit the top and bottom handle portions. One embodiment of such asymmetrical orientation and alignment structures is illustrated in Figures 8, 9, 10, 11 and 12. In this embodiment, the inner stylet hub 28 contains a pair of protrusions 80, each having a different configuration. Each pair of inner stylet hub protrusions 80 interacts uniquely with only one of a corresponding pair of alignment recesses 81 formed as part of the bottom portion recess 38.

Thus, when the top portion 2 of the handle is fitted to the bottom portion 3 of the handle assembly, a specific predetermined insertion and alignment between each of the handle portions is permitted. This is an important feature of the invention, since the fitting of the handle portions to one another can be used to likewise control the rotational orientation of the inner stylet and outer cannula relative to one another. Thus, the likelihood of misalignment or incomplete fitting, or other such "user faults", are reduced or prevented as a result of this feature. Accordingly, a stylet tip configuration can coordinate with the distal tip configuration of the outer cannula. This feature permits biopsy devices to incorporate specific coordinating cannula and stylet configurations into their structure, which can be adapted to facilitate the use of the device for a given procedure. In Figure 11, for example, the angle of the stylet tip coordinates with the angle of the outer cannula tip so that when assembled, the stylet and outer cannula share the same angled position, which in turn facilitates tissue penetration and reduces trauma to the patient at the sampling site. Similarly, a faceted sides of a stylet tip can coordinate with a beveled tip of an outer cannula in a device, such as that depicted in Figures 1 through 5 and 9. As a result of the above features, the precision of the biopsy device is enhanced by virtue of the features of the handle assembly of the invention.

Now referring to Figures 8 and 10, the bottom portion 3 can be structured to secure the proximal end 61 of the outer cannula 60. As illustrated therein, the bottom portion 3 comprises a recess 38 which is adapted to both accommodate the inner stylet hub 28 of the top portion 2 within, as well as accommodate the proximal end 61 of the outer cannula 60. The proximal end 61 of the outer cannula 60 can further comprise an outer cannula hub 62. The outer cannula hub 62 can be removably secured within the bottom portion recess 38 and retained therein by engaging the cap 70 to the bottom portion 3.

In a preferred embodiment, the outer cannula hub 62 can be dimensioned or structured to simultaneously prevent longitudinal (vertical) movement and rotational movement relative to the bottom portion 3 of the handle assembly 1. The outer cannula 60 is permanently attached (by welding metal to metal) to the outer cannula hub 62. One embodiment of the outer cannula hub 62 comprises an overall cylindrical structure with a portion of the outer wall being flat, and a circumscribing step 113 (see Figures 8 and 10). The bottom portion recess 38 can be molded with the corresponding receiving configuration, including a receiving step (not shown) such that when the outer cannula hub 62 resides within the recess 38, neither the outer cannula hub 62 nor the outer cannula 60 can rotate or migrate vertically when the end cap 70 is secured. Collectively, these features create a "mechanical trap" which prevents migration of the cannula hub and outer cannul, which as a result controls the orientation of the outer cannula as well as compensates for the difficulties associated with adhering metal components to plastic ones.

The collective function of the inner stylet hub 28 configuration, bottom portion recess 38 configuration, and outer cannula hub 62 configuration results in the capability of forming a predetermined alignment and arrangement between the inner stylet 50 and the outer cannula 60. Accordingly, a bone biopsy core sampling device can be structured such that the inner stylet 50 can have a faceted tip which coordinates with the edges of the distal tip of the outer cannula 60, which can include a plurality of sharp tips and edges. Similarly, an bone marrow aspiration device can be structured such that an angle cut on the distal tip of the inner stylet 50 can coordinate with an angle cut on the distal tip of the outer cannula 60. Thus, the inner stylet and outer cannula distal ends can be designed to penetrate tissue in a less traumatic manner.

In a preferred embodiment, the outer cannula 60 is composed of metal and permanently fixed to the outer cannula hub 62 also composed of metal. This material arrangement provides material compatibility between the cannula and its hub, and facilitates the coupling of these two components. In even more preferred embodiment, the proximal end 63 of the outer cannula hub 62 is adapted to couple to a syringe or other aspiration device. Thus, the proximal end 63 of the outer cannula hub 62 can comprise luer threading (see Figures 8 and 10) which cooperates with corresponding luer threading on the tip of an aspiration source (not shown). In accordance with this embodiment of the biopsy device, once the top portion 2 and inner stylet 50 are removed after penetration of the device into the bone, a syringe can be coupled to the outer cannula 60 via the cannula hub 62 and bone marrow fluid samples can be withdrawn by using a syringe.

Figures 8 and 9 illustrate the handle assembly of the invention as part of a bone biopsy core sampling device, whereas Figures 10 and 11 illustrate the handle assembly of the invention as part of a bone marrow aspiration device. As can be seen from these figures, the same basic handle assembly components are used with differing inner stylet and outer cannula structures. Referring to Figures 8 and 9, the outer cannula of a bone biopsy core sampling device comprises a serrated, bladed tip to facilitate rotational boring through hard bone tissue.

In another embodiment, the length of the inner stylet hub 28 can be marked with visible indicia 29. As seen in Figures 9 and 10, a depth marking of "1 cm" (one centimeter) is located on the exterior surface of the inner stylet hub 28 to provide the user with a readily visible reference point corresponding to sample length. In use, once the outer cannula 60 has been advanced further into the bone, the inner stylet 50 can be reinserted into the proximal end of the outer cannula 60 such that the user can view the inner stylet hub 28 and, accordingly, the extent to which the inner stylet protrudes proximally relative to the upper surface 31 of the bottom portion 3 of the handle. By viewing thusly, the user can quickly estimate or assess the length of the tissue residing thus far within the distal portion of the outer cannula 60.

In a bone marrow aspiration device such as that depicted in Figures 10 and 11, the inner stylet tip and outer cannula tip are angle-cut, and an adjustable depth guide assembly 100 is shown. In one embodiment, the adjustable depth guide assembly 100 is adapted to removably attach onto the exterior of the cap 70, and comprises two components: a depth guide coupler 101 and depth guide stopper 102. The coupler 101 and stopper 102 are each generally tubular in configuration and removably accommodate the dimensions of the outer cannula to be inserted therethrough. The combined length of the depth guide coupler and depth guide stopper can be movable relative to one another. In a preferred embodiment and as shown in Figures 10 and 11, the depth guide coupler 101 is both threaded and marked with measuring indicia such that the coupler 101 interacts with receiving threads (not shown) in the depth guide stopper 102. The depth guide coupler 101 functions to join the depth guide stopper 102 to the cap 70 of the handle assembly 1. The distal end of the depth guide stopper 102 can further comprise a flattened portion 103. Thus, rotating the coupler 101 and stopper 102 relative to one another controls the penetration depth of the outer cannula 60 when inserted into the patient such that the flattened end of the stopper 102 abuts the exterior surface of the patient preventing further advancement. Accordingly, the user is capable of performing an anatomy-specific procedure based on the dimensions of a particular bone and a particular patient's bone structure. Alternatively, a single-piece depth guide can be used. As a result of this feature, unnecessary tissue damage caused by the outer cannula 60 can be reduced or avoided.

To further enhance grippability of the device, the exterior surface of the handle assembly can comprise friction-enhancing texturing. Suitable friction-enhancing texturing can include, but is not limited to, slight surface roughening. Again, as the inventive features enhance comfort to the user, friction-enhancing texturing which can be used is that which improves tactile contact but does not significantly reduce comfort.

The components of the biopsy device and handle assembly of the invention can be manufactured using techniques and machining equipment readily available to those in the biopsy device manufacturing field. The materials used for the various components of the invention are those which are suitable for use in medical applications and which can maintain their structural integrity when encountering the forces associated with hard tissue biopsy procedures. Preferably, the top portion and bottom portion of the handle assembly can be composed of molded rigid plastic. The adjustable penetration depth guide can also be composed of plastic. The stylet and cannula components, as well as the outer cannula hub, can be composed of metal.

### Example 1 Bone Biopsy Core Sample Procedure

The bone biopsy core sampling device is generally structured with an inner stylet which functions as a trocar and an outer cannula with a sharpened distal tip to facilitate penetration through the bone tissue to obtain the core tissue sample. Once the sampling site has been determined, the assembled device with the handle portions coupled is forced through the tissue until the distal portion of the outer cannula containing the inner stylet reaches the sampling area. At this point, the inner stylet coupled to the top portion of the handle can be removed and the outer cannula further advanced into the tissue by virtue of force exerted by the user on the handle both longitudinally and rotationally. The user can then determine or estimate the sample length by reinsertion of the inner stylet and viewing the extent that the proximal end of the inner stylet extends beyond the upper surface of the bottom portion of the handle assembly. This step can be repeated until the desired sample length has been cored. Once the desired sample length has been obtained, the bottom portion of the handle is pulled proximally and the outer cannula is withdrawn from the tissue. The sample retained within the outer cannula can be expelled using a rod or other similar instrument inserted through the proximal end of the outer cannula.

### Example 2 Bone Marrow Aspiration Procedure

The bone marrow aspiration device is generally structured with an inner stylet which functions as a trocar and an outer cannula which functions as an aspiration cannula when a suction source, such as a syringe, is attached to the proximal end thereof. Once the sampling site has been selected and an anatomical assessment made of the patient's bone geometry and dimensions, the adjustable depth guide assembly is adjusted to the appropriate length. The assembled device with the handle portions coupled is then forced through the tissue until the distal portion of the outer cannula containing the inner stylet reaches the sampling area. Ideally, the distal tip of the outer cannula is positioned within the bone at the location to permits unobstructed fluid ingress into the outer cannula. At this point, the inner stylet coupled to the top portion of the handle can be removed, and an aspiration source such as a syringe is then attached to the proximal hub of the outer cannula. Suction force is applied thus withdrawing the fluid and tissue surrounding the sampling site into the outer cannula. The outer cannula and bottom portion of the handle assembly are withdrawn from the site.

### Industrial Applicability:

The biopsy device handle assembly of the invention can be used in medical procedures where obtaining a tissue sample from relatively hard tissue requires penetration. The invention is particularly applicable in bone biopsy core sampling devices and bone marrow aspiration devices whereby considerable physical force by the user's hand is required during the tissue penetration and sampling steps of the procedure. In such procedures, ergonomics of the handle assembly can enhance the operation of the device entire, and improve the comfort and accuracy of the procedure for both the user and patient.

The invention has been described herein above with reference to various specific and preferred embodiments and techniques. It will be understood by one of ordinary skill that reasonable modifications and variations of such embodiments and techniques can be made without substantially departing from the scope of the invention as defined by the claims set forth below.

## Claims

1. A handle assembly (1) for biopsy devices comprising at least two separable handle components together forming a generally elongated arcuate handle, said handle components comprising:
a top portion (2) having an upper surface (22), circumscribing side surface (23), and lower surface (21) ;
a bottom portion (33) having an upper surface (31), circumscribing side surface (32), and lower surface (33); and
wherein said top portion lower surface mates with said bottom portion upper surface to form an assembled handle comprising a generally arcuate handle wherein the outward curve is at the top portion and the inward curve is at the bottom portion; **characterized in that**
said assembled handle further comprises a plurality of concave indentations (4a-4d) located on both elongated sides of the assembled handle top and bottom portions.

2. The handle assembly according to claim 1, wherein said concave indentations (4a-4d) are positioned such that each of the two longer side regions of the assembled handle comprises two concave indentations positioned apart from one another, and each concave indentation is positioned opposite a corresponding concave indentation on the opposite side of the handle.

3. The handle assembly according to claim 1 or 2, wherein the circumscribing edges (26, 36) of both the top portion (2) and bottom portion (3) at the peripheral juncture at which they mate are rounded, curved and smooth so that the central region of the handle assembly is substantially devoid of sharp edges.

4. The handle assembly according to any one of claims 1 to 3, wherein the bottom portion (3) of the handle assembly comprises a cap (70) adapted to removably attach to the lower surface (33) of the bottom portion (3) of the handle to cover the juncture where an outer cannula (60) can be secured in the handle, and adapted to accommodate a portion of a cannula therethrough.

5. The handle assembly according to claim 4, wherein said cap (70) comprises a generally conical shape.

6. The handle assembly according to any one of the preceding claims, the lower surface (21) of the top portion (2) and the upper surface (31) of the bottom portion (3) of the handle assembly comprise cooperating interfitting alignment structures (120, 37).

7. The handle assembly according to claim 6, wherein said interfitting alignment structures comprise cooperating nub-and-dint structures (120, 37).

8. The handle assembly according to any one of the preceding claims, wherein the lower surface (21) of the top portion (2) of the handle assembly comprises an inner stylet hub (28).

9. The handle assembly according to claim 8 wherein the exterior surface of the inner stylet hub (28) is marked with viewable indicia (29).

10. The handle assembly according to claim 8 or 9 wherein the bottom portion (3) of the handle assembly comprises a recess (28) for receiving the stylet hub.

11. The handle assembly according to any one of the preceding claims, wherein the exterior surface of the handle assembly comprises friction-enhancing texturing.

12. The handle assembly according to claim 1 comprising a total of four concave indentations (4a-4d) having two concave indentations located on each of the longer sides of said handle assembly.

13. A biopsy device comprising:
(a) a handle assembly (1) as claimed in any one of claims 1 to 12;
(b) an inner stylet (50) coupled to said top portion (2) of said handle assembly; and
(c) an outer cannula (60) secured to said bottom portion (3) of said handle assembly,
wherein said inner stylet is removable from within said outer cannula by separating said top portion and bottom portion of said handle assembly.

14. The biopsy device according to claim 13 which is a bone biopsy core sampling device.

15. The biopsy device according to claim 14, wherein said inner stylet (50) comprises a sharp distal tip and said outer cannula (60) comprises a sharp distal tip.

16. The biopsy device according to claim 15, wherein said sharp distal tip comprises a plurality of sharpened points.

17. The biopsy device according to claim 15, wherein said inner stylet distal tip and said outer cannula distal tip coordinate with one another to form a shared faceted surface when the handle assembly of said device is assembled.

18. The biopsy device according to claim 13 which is a bone marrow aspiration device.

19. The biopsy device according to claim 18, wherein said inner stylet (50) comprises an angle cut sharpened distal tip and said outer cannula (60) comprises an angle cut sharpened distal tip, and wherein said distal tips coordinate with one another to form a shared tip angle when the handle assembly (1) of said device is assembled.

20. The biopsy device according to claim 18 or 19 further comprising an adjustable depth guide assembly (100).

21. The biopsy device according to claim 20, wherein said adjustable depth guide assembly (100) is adapted to removably couple to the bottom portion (3) of said handle assembly (1).

22. The biopsy device according to claim 20 or 21, wherein said adjustable depth guide assembly (100) comprises a depth guide coupler (101) and depth guide stopper (102) wherein said coupler and stopper are movably coupled to one another in such a manner as to permit longitudinal adjustment.

## Patentansprüche

1. Griffanordnung (1) für Biopsievorrichtungen, die mindestens zwei trennbare Griffkomponenten aufweist, die gemeinsam einen allgemein langgestreckten bogenförmigen Griff bilden, wobei die Griffkomponenten Folgendes aufweisen:
einen oberen Bereich (2), der eine obere Oberfläche (22), eine umschreibende Seitenfläche (23) und eine untere Oberfläche (21) hat;
einen unteren Bereich (3), der eine obere Oberfläche (31), eine umschreibende Seitenfläche (32) und eine untere Oberfläche (33) hat; und
wobei die untere Oberfläche des oberen Bereichs mit der oberen Oberfläche des unteren Bereichs zusammenpasst unter Bildung eines zusammengesetzten Griffs, der einen allgemein bogenförmigen Griff aufweist, wobei sich die Auswärtskrümmung an dem oberen Bereich und die Einwärtskrümmung an dem unteren Bereich befindet; **dadurch gekennzeichnet, dass**
der zusammengesetzte Griff ferner eine Vielzahl von konkaven Vertiefungen (4a bis 4d) aufweist, die an beiden langgestreckten Seiten des oberen und des unteren Bereichs des zusammengesetzten Griffs positioniert sind.

2. Griffanordnung nach Anspruch 1, wobei die konkaven Vertiefungen (4a bis 4d) so positioniert sind, dass jeder der zwei längeren Seitenbereiche des zusammengesetzten Griffs zwei konkave Vertiefungen aufweist, die voneinander entfernt positioniert sind, und jede konkave Vertiefung einer entsprechenden konkaven Vertiefung an der entgegengesetzten Seite des Griffs gegenüberliegend positioniert ist.

3. Griffanordnung nach Anspruch 1 oder 2, wobei die umschreibenden Ränder (26, 36) sowohl des oberen Bereichs (2) als auch des unteren Bereichs (3) an der peripheren Verbindungsstelle, an der sie zusammengefügt sind, gerundet, gekrümmt und glatt sind, so dass der Mittelbereich der Griffanordnung im Wesentlichen frei von scharfen Kanten ist.

4. Griffanordnung nach einem der Ansprüche 1 bis 3, wobei der untere Bereich (3) der Griffanordnung eine Kappe (70) aufweist, die ausgebildet ist, um an der unteren Oberfläche (33) des unteren Bereichs (3) des Griffs abnehmbar angebracht zu werden, um die Verbindungsstelle abzudecken, an der eine äußere Kanüle (60) in dem Griff befestigt werden kann, und ausgebildet ist, um einen Bereich einer Kanüle durch sich hindurch aufzunehmen.

5. Griffanordnung nach Anspruch 4, wobei die Kappe (70) eine allgemein konische Gestalt aufweist.

6. Griffanordnung nach einem der vorhergehenden Ansprüche, wobei die untere Oberfläche (21) des oberen Bereichs (2) und die obere Oberfläche (31) des unteren Bereichs (3) der Griffanordnung zusammenwirkende, ineinandergreifende Ausfluchtungsausbildungen (120, 37) aufweisen.

7. Griffanordnung nach Anspruch 6, wobei die ineinandergreifenden Ausfluchtungsausbildungen zusammenwirkende Noppen-und-Dellen-Ausbildungen (120, 37) aufweisen.

8. Griffanordnung nach einem der vorhergehenden Ansprüche, wobei die untere Oberfläche (21) des oberen Bereichs (2) der Griffanordnung eine innere Stylettnabe (28) aufweist.

9. Griffanordnung nach Anspruch 8, wobei die äußere Oberfläche der inneren Stylettnabe (28) mit sichtbaren Markierungen (29) markiert ist.

10. Griffanordnung nach Anspruch 8 oder 9, wobei der untere Bereich (3) der Griffanordnung eine Ausnehmung (38) zur Aufnahme der Stylettnabe aufweist.

11. Griffanordnung nach einem der vorhergehenden Ansprüche, wobei die äußere Oberfläche der Griffanordnung eine die Reibung verstärkende Texturierung aufweist.

12. Griffanordnung nach Anspruch 1, die insgesamt vier konkave Vertiefungen (4a bis 4d) aufweist, wobei zwei konkave Vertiefungen an jeder der längeren Seiten der Griffanordnung angeordnet sind.

13. Biopsievorrichtung, die Folgendes aufweist:
(a) eine Griffanordnung (1) nach einem der Ansprüche 1 bis 12;
(b) ein inneres Stylett (50), das mit dem oberen Bereich (2) der Griffanordnung verbunden ist; und
(c) eine äußere Kanüle (60), die an dem unteren Bereich (3) der Griffanordnung befestigt ist,
wobei das innere Stylett aus dem Inneren der äußeren Kanüle durch Trennen des oberen Bereichs und des unteren Bereichs der Griffanordnung entfernbar ist.

14. Biopsievorrichtung nach Anspruch 13, die eine Knochenbiopsie-Kernprobenahmevorrichtung ist.

15. Biopsievorrichtung nach Anspruch 14, wobei das innere Stylett (50) eine scharfe distale Spitze aufweist und die äußere Kanüle (60) eine scharfe distale Spitze aufweist.

16. Biopsievorrichtung nach Anspruch 15, wobei die scharfe distale Spitze eine Vielzahl von geschärften Stellen aufweist.

17. Biopsievorrichtung nach Anspruch 15, wobei die distale Spitze des inneren Styletts und die distale Spitze der äußeren Kanüle miteinander koordiniert sind, um eine gemeinsame facettierte Oberfläche zu bilden, wenn die Griffanordnung der Vorrichtung zusammengesetzt ist.

18. Biopsievorrichtung nach Anspruch 13, die eine Knochenmark-Aspirationsvorrichtung ist.

19. Biopsievorrichtung nach Anspruch 18, wobei das innere Stylett (50) eine winkelschnitt-geschärfte distale Spitze aufweist und die äußere Kanüle (60) eine winkelschnitt-geschärfte distale Spitze aufweist, und wobei die distalen Spitzen miteinander koordiniert sind, um einen gemeinsamen Spitzenwinkel zu bilden, wenn die Griffanordnung (1) der Vorrichtung zusammengesetzt ist.

20. Biopsievorrichtung nach Anspruch 18 oder 19, die ferner eine einstellbare Tiefenführungsanordnung (100) aufweist.

21. Biopsievorrichtung nach Anspruch 20, wobei die einstellbare Tiefenführungsanordnung (100) ausgebildet ist, um mit dem unteren Bereich (3) der Griffanordnung (1) abnehmbar verbunden zu werden.

22. Biopsievorrichtung nach Anspruch 20 oder 21, wobei die einstellbare Tiefenführungsanordnung (100) ein Tiefenführungskoppelelement (101) und ein Tiefenführungsstoppelement (102) aufweist, wobei das Koppelelement und das Stoppelement bewegbar so miteinander gekoppelt sind, dass sie eine Einstellung in Längsrichtung zulassen.

## Revendications

1. Ensemble poignée (1) pour dispositifs de biopsie, comprenant au moins deux composants de poignée séparables formant ensemble une poignée courbe sensiblement allongée, les dits composants de poignée comprenant :
une partie supérieure (2) ayant une surface supérieure (22), une surface latérale d'entourage (23) et une surface inférieure (21) ;
une partie inférieure (3) ayant une surface supérieure (31), une surface latérale d'entourage (32) et une surface inférieure (33) ; et
dans lequel la dite surface inférieure de la partie supérieure s'accouple avec la dite surface supérieure de la partie inférieure pour former une poignée assemblée comprenant une poignée sensiblement arquée dans laquelle la courbure vers l'extérieur est à la partie supérieure et la courbure vers l'intérieur est à la partie inférieure ,
**caractérisé en ce que** la dite poignée assemblée comprend en outre une pluralité d'indentations concaves (4a-4d) sur les deux côtés allongés des parties supérieure et inférieure de la poignée assemblée.

2. Ensemble poignée selon la revendication 1, dans lequel les dites indentations concaves (4a-4d) sont situées de sorte que chacune des deux plus longues régions latérales de la poignée assemblée comprend deux indentations concaves espacées l'une de l'autre, et chaque indentation concave est située à l'opposé d'une indentation concave correspondante sur le côté opposé de la poignée.

3. Ensemble poignée selon la revendication 1 ou 2, dans lequel les bords d'entourage (26, 36) à la fois de la partie supérieure (2) et de la partie inférieure (3), à l'endroit de la jonction périphérique où elles s'accouplent, sont arrondis, courbes et lisses de sorte que la région centrale de l'ensemble poignée soit sensiblement exempte d'arêtes vives.

4. Ensemble poignée selon une quelconque des revendications 1 à 3, dans lequel la partie inférieure (3) de l'ensemble poignée comprend un capuchon (70) prévu pour fixation séparable à la surface inférieure (33) de la partie inférieure (3) de la poignée de manière à couvrir la jonction où une canule extérieure (60) peut être fixée dans la poignée, et prévu pour recevoir une partie d'une canule traversante.

5. Ensemble poignée selon la revendication 4, dans lequel le dit capuchon (70) présente une forme sensiblement conique.

6. Ensemble poignée selon une quelconque des revendications précédentes, dans lequel la surface inférieure (21) de la partie supérieure (2) et la surface supérieure (31) de la partie inférieure (3) de l'ensemble poignée comportent des structures d'alignement coopérantes en interconnexion (120, 37).

7. Ensemble poignée selon la revendication 6, dans lequel les dites structures d'alignement en interconnexion comprennent des structures à bouton et creux coopérantes (120, 37).

8. Ensemble poignée selon une quelconque des revendications précédentes, dans lequel la surface inférieure (21) de la partie supérieure (2) de l'ensemble poignée comprend un moyeu de stylet intérieur (28).

9. Ensemble poignée selon la revendication 8, dans lequel la surface extérieure du moyeu de stylet intérieur (28) comporte des marques visibles (29).

10. Ensemble poignée selon la revendication 8 ou 9, dans lequel la partie inférieure (3) de l'ensemble poignée comporte un évidement (38) pour recevoir le moyeu de stylet.

11. Ensemble poignée selon une quelconque des revendications précédentes, dans lequel la surface extérieure de l'ensemble poignée présente une texture renforçant la friction.

12. Ensemble poignée selon la revendication 1, comprenant un total de quatre indentations concaves (4a-4d) ayant deux indentations concaves situées sur chacun des grands cotés du dit ensemble poignée.

13. Dispositif de biopsie comprenant :
a) un ensemble poignée (1) selon une quelconque des revendications 1 à 12,
b) un stylet intérieur (50) couplé à la dite partie supérieure (2) du dit ensemble poignée, et
(c) une canule extérieure (60) fixée à la dite partie inférieure (3) du dit ensemble poignée,
dans lequel le dit stylet intérieur est démontable de l'intérieur de la dite canule extérieure par séparation de la dite partie supérieure et de la dite partie inférieure du dit ensemble poignée.

14. Dispositif de biopsie selon la revendication 13, qui est un dispositif d'échantillonnage pour biopsie osseuse.

15. Dispositif de biopsie selon la revendication 14, dans lequel le dit stylet intérieur (50) présente une extrémité distale pointue et la dite canule extérieure (60) présente une extrémité distale pointue

16. Dispositif de biopsie selon la revendication 15, dans lequel la dite extrémité distale pointue comprend une pluralité de pointes aigües.

17. Dispositif de biopsie selon la revendication 15, dans lequel la dite extrémité distale du stylet intérieur et la dite extrémité distale de la canule extérieure sont mutuellement coordonnées pour former une surface à facettes partagées lorsque l'ensemble poignée du dit dispositif est assemblé.

18. Dispositif de biopsie selon la revendication 13, qui est un dispositif d'aspiration de moelle osseuse.

19. Dispositif de biopsie selon la revendication 18, dans lequel le dit stylet intérieur (50) comprend une extrémité distale coupée à angle vif et la dite canule extérieure (60) comprend une extrémité distale coupée à angle vif, et dans lequel les dites extrémités distales sont mutuellement coordonnées pour former un angle d'extrémité partagé lorsque l'ensemble poignée (1) du dit dispositif est assemblé.

20. Dispositif de biopsie selon la revendication 18 ou 19, comprenant en outre un dispositif de guidage de profondeur réglable (100).

21. Dispositif de biopsie selon la revendication 20, dans lequel le dit dispositif de guidage de profondeur réglable (100) est prévu pour s'accoupler de façon séparable à la partie inférieure (3) du dit ensemble poignée (1).

22. Dispositif de biopsie selon la revendication 20 ou 21, dans lequel le dit dispositif de guidage de profondeur réglable (100) comprend un coupleur de guidage de profondeur (101) et une butée de guidage de profondeur (102), et dans lequel le dit coupleur et la dite butée sont reliés l'un à l'autre de manière mobile afin de permettre un réglage longitudinal.
